# EUROPÄISCHE PATENTSCHRIFT

(11) **EP 1 068 232 B1**
(45) Veröffentlichungstag und Bekanntmachung des Hinweises auf die Patenterteilung: **23.03.2005**
(21) Anmeldenummer: 00904996.6
(22) Anmeldetag: 01.02.2000
(51) Int. Cl.: C07K 14/47, C12N 15/12, C12N 9/22

(54) **HUMANES ANTIBIOTISCHES PROTEIN**
HUMAN ANTIBIOTIC PROTEIN
PROTEINE ANTIBIOTIQUE HUMAINE

(30) Priorität: 01.02.1999 DE 19905128; 08.10.1999 DE 19949436
(43) Veröffentlichungstag der Anmeldung: 17.01.2001
(73) Patentinhaber: Schering Aktiengesellschaft, 13353 Berlin (DE)
(72) Erfinder: CHRISTOPHERS, Enno, Prof. Dr., 24105 Kiel (DE); HARDER, Jürgen, 24105 Kiel (DE); SCHRÖDER, Jens, Prof. Dr., 24241 Blumenthal (DE)
(74) Vertreter: Dörries, Hans Ulrich, Dr.
(86) Internationale Anmeldenummer: PCT/EP2000/000776
(87) Internationale Veröffentlichungsnummer: WO 2000/046245

(56) Entgegenhaltungen:
- EP-A- 0 943 679
- WO-A-94/15561
- WO-A-99/13080
- HARDER J: "A PEPTIDE ANTIBIOTIC FROM HUMAN SKIN" NATURE,GB,MACMILLAN JOURNALS LTD. LONDON, Bd. 387, 26. Juni 1997 (1997-06-26), Seite 861 XP002072639 ISSN: 0028-0836 in der Anmeldung erwähnt
- J-M SCHRÖDER: "Identification and structural characterization of chemokines in lesional skin material of patients with inflammatory skin disease" METHODS IN ENZYMOLOGY, Bd. 268, 1998, Seiten 266-296, XP000864768 SAN DIEGO US in der Anmeldung erwähnt
- ROSENBERG H F ET AL: "MOLECULAR CLONING AND CHARACTERIZATION OF A NOVEL HUMAN RIBONUCLEASE (RNASE K6): INCREASING DIVERSITY IN THE ENLARGING RIBONUCLEASE GENE FAMILY" NUCLEIC ACIDS RESEARCH,GB,OXFORD UNIVERSITY PRESS, SURREY, Bd. 24, Nr. 18, 1996, Seiten 3507-3513, XP002074516 ISSN: 0305-1048

## Beschreibung

Die Erfindung betrifft Proteine / Peptide (Proteine), die antibiotisch wirksam sind. Weiterhin umfaßt die Erfindung ein Verfahren zur Reinigung von bestimmten antibiotischen Proteinen. Darüber hinaus bezieht sich die Erfindung auf eine Verwendung der Proteine zur antibiotischen Behandlung oder auf eine Verwendung von Zellen, welche mit einer DNA transfiziert wurden, die für die antibiotischen Proteine kodiert.

### STAND DER TECHNIK:

Pathogene Mikroorganismen befinden sich gewöhnlich auf der Oberfläche von Epithelien. Dort haften sie an und vermehren sich. Gelegentlich dringen sie auch in tiefere Gewebsschichten ein. Da die Immunantwort gegen diese pathogenen Mikroorganismen langsam einsetzt, ist es nicht verwunderlich, daß die Epithelzellen über eine Abwehr verfügen, mit Hilfe von sezernierten antimikrobiellen Substanzen gegen die Mikroorganismen vorzugehen. Einige dieser Substanzen führen zu einer Mangelernährung bei den Mikroorganismen, andere töten die Mikroorganismen ab, indem Strukturen der Mikroorganismen zerstört werden.

Die Epithelien von Säugern sind normaler Weise nicht infiziert. Dennoch ist die Hautoberfläche von Bakterien und Pilzen dicht besiedelt. Es handelt sich dabei um haut - spezifische Mikroorganismen, die, wenn sie unter Kontrolle stehen, nicht pathogen sind.

Das erste bekannte, epitheliale j' - Defensin, das die Luftröhre der Rinder schützt, ist TAP, welches 64 Aminosauren besitzt. (D.G. ZASLOFF et al. (1991) Proc. Natl. Acad. Sci. USA, Vol. 88. p 3952 - 3956). Dieses TAP vom Rind wirkt anti - bakteriell gegen *E. coli. Klebsiella pneumoniae, Staphylococcus aureus* und *Pseudomonas aeruginosa* bei einer minimalen Inhibitionskonzentration von 12 - 50 µg/ml. Auch *Candida albicans* wird zerstört. Hierbei handelt es sich um ein Protein, welches gewebespezifisch exprimiert wird.

Ein weiteres β - Defensin schützt die Zunge von Rindern, nämlich LAP, welches eine hohe Homologie zu TAP aufweist. (B.S. SCHONWETTER et al. (1995) Science Vol. 267, p 1645 - 1648)

Erst 1995 wurde das erste humane j' - Defensin gefunden, welches hBD-1 genannt wird. (K.W. BENSCH et al (1995) FEBS Lett, Vol. 368, p 331 - 335) So besitzt hBD-1 eine anti - bakterielle Wirkung gegen Gram - negative Bakterien bei einer Konzentration von 60 bis 500 µg/ml. (M. GOLDMAN et al. (1997) Cell. Vol. 88, p 553 - 560) hBD-1 wird dominant in den Nieren exprimiert, jedoch auch andere epitheliale Gewebe sekretieren hBD-1.

Unabhängig von diesen Untersuchungen war von Interesse, was die Haut normalerweise gesund hält und warum die Haut selten infiziert wird. Das zweite beim Menschen isolierte β - Defensin war das hBD-2, das aus 41 Aminosäuren besteht. (J. HARDER et al. (1997) Nature, Vol. 387, p 861) hBD-2 wirkte sehr effektiv gegen Gram - negative Bakterien mit einer LD₉₀ von 10 µg/ml, wogegen bei Gram - positiven Bakterien der Wert bei über 100 µg/ml liegt. Das hBD-2 ist ein induzierbares Peptid, welches selbst durch einige hitzeinaktivierte Bakterien induziert werden kann.

Ein weiteres humanes antibiotisches Protein ist das ALP, ein Proteaseinhibitor (J.A. KRAMPS et al. (1988) Biol. Chem. Hoppe Seyler, Vol 369, p 83 - 87), das von Keratinozyten produziert wird und sich gegen einige Bakterien und Pilze richtet.

Die Angriffe der antibiotischen Protein sind sehr unterschiedlich. Eine Interaktion mit der Membran der Mikroorganismen sind üblich. Lipophile Strukturen vieler antibiotischer Protein und der Defensine sprechen für ein Interkalieren in den Membranen oder ein Penetrieren der Membranen. Die antimikrobiellen Proteine und Peptide wirken erst in den Mikroorganismen selbst toxisch.

### AUFGABEN UND LÖSUNG:

Aufgabe der Erfindung ist es, weitere humane, antibiotische Proteine und deren Derivate anzubieten, welche wirksam gegen Mikroorganismen, insbesondere gegen Gram - negative und Gram - positive Bakterien, gegen Pilze und gegen Viren einsetzbar sind.

### SEQUENZEN DER REIFEN PROTEINE

Die Aufgabe wird gelöst durch mindestens ein Protein,
a) das als aktives, reifes Protein / Peptid (Protein) einer der folgenden Sequenzen aufweist:
   (i) SEQ ID NO: 1 (Sequenz Protokoll Nr. 1) (SAP-2);
   oder
b) das als aktives, reifes Protein allelische Modifikationen der zuvor unter a) genannten Aminosäure - Sequenz aufweist, wobei mindestens eine Aminosäure der Aminosäure - Sequenz substituiert, deletiert oder insertiert ist, wobei die antimikrobielle und / oder antibiotische Aktivität des unter b) definierten aktiven, reifen Proteins erhalten bleibt.

Vorteilhaft ist ein erfindungsgemäßes Protein,
das antimikrobiell oder antibiotisch wirksam ist und
das eine Mobilität von 6 kDa in der SDS - Gelelektrophorese aufweist.

Mehr bevorzugt ist ein erfindungsgemäßes Protein, das eine antibiotische Aktivität gegen *Escherichia coli* oder *Staph. aureus* in einer Konzentration von weniger als 100 µg/ml aufweist.

In der Literatur soll in Zukunft die Bezeichnung SAP-2 durch RNase 7 ersetzt werden.

Sehr bevorzugt ist ein erfindungsgemäßes Protein, welches ein mit der humanen Aminosäure - Sequenz versehenes Protein ist (vgl. SEQ ID NO: 1).

### Zu SEQ ID NO: 1

Alle allelischen Modifikationen, die die Substitutionen, die Deletionen und/oder die Insertionen von bis zu 30 Aminosäuren umfassen, gehören zur Gruppe der erfindungsgemäßen Proteine des SEQ ID NO: 1. Bevorzugt sind Deletionen, Substitutionen und/oder Insertionen von bis zu 20 Aminosäuren, mehr bevorzugt von bis zu 10 Aminosäuren, am meisten bevorzugt sind die Deletionen, Substitutionen und / oder Insertionen von einer, zwei, drei, vier, fünf, sechs, sieben, acht oder neun Aminosäuren. Die allelischen Modifikationen sind nicht auf die natürlich vorkommenden Allele begrenzt, vielmehr sind auch im Labor entstandene (nicht in der Natur selbst vorkommende) Veränderungen der Aminosäure - Sequenz möglich.

### SEQUENZEN DER REIFEN PROTEINE MIT SIGNALSEQUENZ

Die Aufgabe wird weiterhin gelöst durch mindestens ein Protein, das eine Signalsequenz und ein reifes erfindungsgemäßes Protein umfaßt,
d) wobei das Protein folgende Sequenz aufweist:
   (i) SEQ ID NO:2 (PreSAP-2);
   oder
e) wobei das Protein allelische Modifikationen der zuvor unter d) genannten Aminosäure - Sequenz aufweist, wobei wenigstens eine Aminosäure der Aminosäure - Sequenz substituiert, deletiert oder insertiert ist, wobei die antimikrobielle und / oder antibiotische Aktivität des unter e) definierten aktiven, reifen Proteins erhalten bleibt.

### Zu SEQ ID NO:2

Alle allelischen Modifikationen, die die Substitutionen, die Deletionen und/oder die Insertionen von bis zu 35 Aminosäuren umfassen, gehören zur Gruppe der erfindungsgemäßen Proteine des SEQ ID NO: 2 Bevorzugt sind Deletionen, Substitutionen und/oder Insertionen von bis zu 23 Aminosäuren, mehr bevorzugt von bis zu 12 Aminosäuren, am meisten bevorzugt sind die Deletionen, Substitutionen und / oder Insertionen von einer, zwei, drei, vier, fünf, sechs, sieben, acht oder neun Aminosäuren. Die allelischen Modifikationen sind nicht auf die natürlich vorkommenden Allele begrenzt, vielmehr sind auch im Labor entstandene (nicht in der Natur selbst vorkommende) Veränderungen der Aminosäure - Sequenz möglich.

Am meisten bevorzugt ist ein erfindungsgemäßes Protein, das ein rekombinantes Protein ist. Dabei können die Proteine glykosiliert sein, wenn es sich um SAP-2 oder eine Variante davon handelt.

Die erfindungsgemäßen Proteine umfassen die reifen Proteine und die entsprechenden Vorläuferproteine, welche sich aus einer Signalsequenz und der Sequenz des reifen Proteins zusammensetzen. Dabei geht die Signalsequenz der Sequenz des reifen Proteins voraus. Das reife Protein beginnt mit der zuvor genannten N - terminalen Sequenz unter Punkt a). Die Signalsequenz ist für die Durchdringung des endoplasmatischen Retikulums erforderlich

Ebenfalls ist es möglich, an den N - Terminus und / oder C - Terminus Schutzgruppen zu synthetisieren, welche aus der Peptidchemie bekannt sind.
Die **Schutzgruppe** des N - Terminus kann bestehen aus:
Alkyl-, Aryl-, Alkylaryl-, Aralkyl-, Alkylcarbonyl- oder Arylcarbonylgruppen mit 1 bis 10 Kohlenstoffatomen, bevorzugt sind Naphthoyl-, Naphthylacetyl-, Naphthylpropionyl-, Benzoylgruppe oder einer Acylgruppe mit 1 bis 7 Kohlenstoffatomen.

Die **Schutzgruppe** des C - Terminus kann bestehen aus:
Einer substituierte oder unsubstituierte Alkoxy- oder Aryloxygruppe mit 1 bis 10 Kohlenstoffatomen oder aus einer Aminogruppe.

Weitere **Schutzgruppen** - sowohl für den N - Terminus als auch für den C - Terminus - sind in Houben-Weyl (1974) Georg Thieme Verlag, 4. Auflage beschrieben. Die Beschreibung der Schutzgruppen in der zitierten Literaturangabe ist Teil der Offenbarung.

Die Sequenz des erfindungsgemäßen Protein kann am N - terminalen und / oder C - terminalen Ende an Stelle einer Schutzgruppe mit weiteren **Rahmen - Aminosäure - Sequenzen** (analog zu der Definition von "frame work" bei Antikörpern) verbunden sein. Diese weiteren Rahmen - Aminosäure - Sequenzen sind für die Bindung des erfindungsgemäßen Protein nicht wesentlich, sie können jedoch Träger von anderen Funktionen sein, so zum Beispiel Chelate oder auch zytostatische oder zytotoxische Sequenzen umfassen. Derartige Rahmen - Aminosäure - Sequenzen treten in der Natur auf. Es kann sich dabei zum Beispiel um die zwischen den hypervariablen Bereichen angeordneten Sequenzen des variablen Bereichs eines Antikörper handeln. Diese Sequenzen werden als "Frame - Work" (Rahmensequenzen) bezeichnet. Als Rahmen - Aminosäure - Sequenzen sind weiterhin nicht abgespaltene Teil- Signalsequenzen eines sezernierten eukaryotischen Proteins bekannt, wobei das Protein in einem Bakterium exprimiert wird. Derartige Signalsequenzen haben bisweilen keinen Einfluß auf die Funktion des nachfolgenden Proteins. Ebenfalls ist es möglich, erfindungsgemäße Proteine hintereinander zu koppeln, wobei Rahmen - Aminosäure - Sequenzen zwischen den Einzelsequenzen angeordnet sind.

Um im Einzelfall zu entscheiden. ob ein bestimmtes erfindungsgemäßes Protein mit wenigstens einer Rahmen - Aminosäure - Sequenz und/oder wenigstens einer Schutzgruppe zum Gegenstand der Erfindung zählt, ist ein Vergleich zwischen
(i) diesem Protein mit Rahmen - Aminosäure - Sequenz und/oder mit Schutzgruppe und
(ii) demselben Protein ohne Rahmen - Aminosäure - Sequenz und ohne Schutzgruppe
anzustellen. Dabei sollten beide verglichenen Moleküle im wesentlichen dieselben Funktionen der Inhibierung oder Bindung aufweisen.

### CDNA ODER DNA KODIEREND FOR DIE ERFINDUNGSGEMÄßEN PROTEINE

Die Erfindung umfaßt weiterhin auch eine cDNA oder DNA,
aa) wobei die cDNA oder DNA eine der folgenden Aminosäure - Sequenzen kodiert:
   (i) SEQ ID NO: 1 (SAP-2);oder
   (iii) SEQ ID NO: 2 (PreSAP-2);
   oder
bb) wobei die cDNA oder DNA allelische Modifikationen einer der Aminosäure - Sequenzen unter aa) kodiert,
worin wenigstens eine Aminosäure der Aminosäure - Sequenz substituiert, deletiert oder insertiert ist,
wobei die antimikrobielle und / oder antibiotische Aktivität des aktiven, reifen Proteins erhalten bleibt.

Bevorzugt sind cDNA und DNA, die ein reifes erfindungsgemäßes Protein kodieren.

Die allelischen Modifikationen sind zuvor unter dem Punkt "Sequenzen der reifen Proteine" definiert worden.

### Zu SEQ ID NO: 1 und 2

Alle allelischen Modifikationen, die die Substitutionen, die Deletionen und/oder die Insertionen von bis zu 90 Nukleotiden umfassen, gehören zur Gruppe der erfindungsgemäßen DNAs der SEQ ID NO: 1 und 2. Bevorzugt sind Deletionen, Substitutionen und/oder Insertionen von bis zu 60 Nukleotiden, mehr bevorzugt von bis zu 30 Nukleotiden, am meisten bevorzugt sind die Deletionen, Substitutionen und / oder Insertionen von einer, zwei, drei, vier, fünf, sechs, sieben, acht oder neun oder 10 bis 29 Nukleotiden. Die allelischen Modifikationen sind nicht auf die natürlich vorkommenden Allele begrenzt, vielmehr sind auch im Labor entstandene (nicht in der Natur selbst vorkommende) Veränderungen der Aminosäure - Sequenz möglich.

Weiterhin umfaßt die Erfindung eine cDNA oder DNA,
cc) wobei die cDNA oder DNA folgende Nukleotidsequenz aufweist:
   (i) SEQ ID NO: 3 (cDNA-SAP-2);
   oder
dd) wobei die cDNA oder DNA eine allelische Modifikation der Nukleotidsequenz unter cc) aufweist, wobei wenigstens ein Nukleotid substituiert, deletiert oder insertiert ist,
wobei die antimikrobielle und / oder antibiotische Aktivität des Proteins, das von der allelischen Modifikation der Nukleotidsequenz unter cc) kodiert wird, erhalten bleibt.

### Zu SEQ ID NO:3

Alle allelischen Modifikationen, die die Substitutionen, die Deletionen und/oder die Insertionen von bis zu 90 Nukleotiden umfassen, gehören zur Gruppe der erfindungsgemäßen DNAs des SEQ ID NO:3. Bevorzugt sind Deletionen, Substitutionen und/oder Insertionen von bis zu 60 Nukleotiden, mehr bevorzugt von bis zu 30 Nukleotiden, am meisten bevorzugt sind die Deletionen, Substitutionen und / oder Insertionen von einer, zwei, drei, vier, fünf, sechs, sieben, acht oder neun oder 10 bis 29 Nukleotiden. Die allelischen Modifikationen sind nicht auf die natürlich vorkommenden Allele begrenzt.

Bevorzugt sind cDNA und DNA, die ein erfindungsgemäßes Protein kodieren.

Eine weitere Ausführungsform der Erfindung umfaßt eine cDNA oder DNA,
ee) wobei die cDNA oder DNA folgende Nukleotidsequenz aufweist:
   (i) SEQ ID NO: 4 (cDNA-PreSAP-2)
   oder
ff) wobei die cDNA oder DNA eine allelische Modifikation einer der Nukleotidsequenzen unter ee) aufweist, wobei wenigstens ein Nukleotid substituiert, deletiert oder insertiert ist, wobei die antimikrobielle und / oder antibiotische Aktivität des Proteins, das von der allelischen Modifikation der Nukleotidsequenz unter ee) kodiert wird, erhalten bleibt. Bevorzugt sind cDNA und DNA, die ein erfindungsgemäßes Präprotein kodieren.

### Zu SEQ ID NO: 4

Alle allelischen Modifikationen, die die Substitutionen, die Deletionen und/oder die Insertionen von bis zu 90 Nukleotiden umfassen, gehören zur Gruppe der erfindungsgemäßen DNAs des SEQ ID NO: 4. Bevorzugt sind Deletionen, Substitutionen und/oder Insertionen von bis zu 60 Nukleotiden, mehr bevorzugt von bis zu 30 Nukleotiden, am meisten bevorzugt sind die Deletionen, Substitutionen und / oder Insertionen von einer, zwei, drei, vier, fünf, sechs, sieben, acht oder neun oder 10 bis 29 Nukleotiden. Die allelischen Modifikationen sind nicht auf die natürlich vorkommenden Allele begrenzt.

Alle DNA-Konstrukte zählen auch dann zu den aufgezählten erfindungsgemäßen Sequenzen, wenn solche Nukleotide ausgetauscht werden, die aufgrund des degenerierten Kodes dieselbe Aminosäure kodieren. Der Austausch derartiger Nukleotide ist offensichtlich und die sich entsprechenden Aminosäuren sind in jedem Biochemie - Lehrbuch offenbart. (R. KNIPPERS, 1982, 3. Auflage, Molekulare Genetik, Georg Thieme Verlag)

Die allelische Modifikationen sind zuvor definiert worden.
Wenn die Aktivität des Proteins angegeben wird, um festzustellen, ob die allelische Modifikation unter die Gruppe der erfindungsgemäßen Proteine zählt, so ist immer das reife Protein zu messen, auch wenn die Signalsequenz ebenfalls angeführt ist. Sollte die Signalsequenz angegeben sein, so ist die Funktion immer an dem Protein zu messen, das nach Entfernen der Signalsequenz erhalten wird.
Die Aktivität der erfindungsgemäßen Proteine mißt sich an seiner Funktion, die eine antibiotische Wirkung, eine antimikrobielle Wirkung, und / oder die Bindung an gegen das reife humane Protein gerichtete Antikörper oder Bindungsmoleküle sein kann.

Weiterhin umfaßt die Erfindung Antikörper oder Fragmente der Antikörper, die Domänen auf dem reifen, erfindungsgemäßen Protein spezifisch erkennen. Wenn das gereinigte erfindungsgemäße Protein vorliegt, ist es für den Fachmann leicht möglich, monoklonale Antikörper herzustellen. Dabei wird die bekannte Methode von Köhler und Milstein und deren Weiterführungen angewendet. Im Einzelnen wird in konventioneller Methode eine Maus mit dem gereinigten Protein mehrfach immunisiert, die Milzzellen entnommen und mit geeigneten Tumorzellen fusioniert. Die Hybride werden anschließend selektioniert. Die Bindungsmoleküle können als Diagnostikum eingesetzt werden, um zum Beispiel festzustellen, ob der jeweilige Patient an einem Mangel oder einer Variante der erfindungsgemäßen Proteine leidet.

Die Protein der Erfindung können zum Beispiel aus Homschuppen von Psoriasis Patienten isoliert werden. Die Reinigung erfolgt gemäß der Beispiele. Die Proteine haben die zuvor beschriebene Aminosäure - Sequenzen. Sie haben ein Molekulargewicht von ca. 20000 ± 2000 bei SAP - 2 (siehe Beispiele). Der isoelektrische Punkt liegt im Bereich von pH 8,5 bis 10,5, wenn die im Beispiel beschriebene Methode angewendet wird. Die erfindungsgemäßen Proteine können natürlichen Ursprungs sein. Die Proteine werden gewonnen, indem sie gemäß der Beispiele geemtet und aufgearbeitet werden. Der Homschuppenüberstand wird gereinigt und die erfindungsgemäßen Proteine isoliert und angereichert. Alle Anreicherungsstufen der Isolierung und der Reinigung sind Teil der Erfindung. Bevorzugt sind die Anreicherungsstufen der Isolierung und Reinigung, bei denen die erfindungsgemäßen Proteine zu pharmazeutischen Zwecken verwendet werden können. So werden Reinigungen von 50 % der Proteine bezogen auf das Gesamtprotein erzielt, bevorzugt sind 85 %, mehr bevorzugt 95 % und am meisten bevorzugt 99 % der Proteine bezogen auf das Gesamtprotein.

Ebenso ist es möglich, die erfindungsgemäßen Proteine synthetisch herzustellen. Dazu zählt die Proteinsynthese nach J.M. SEWART and J.D. YOUNG, San Francisco, 1969 and J. MEIENHOFER, Hormonal Proteins and Peptides Vol. 2 p 46, Academic Press (New York), 1973 und E. SCHODER and K. LUBKE, The Peptides, Vol. 1, Academic Press (New York) 1965. Die Zitate sind Teil der Offenbarung.
Zu den synthetisch hergestellten Proteinen zählen auch die rekombinanten Proteine, die nach bekannten Verfahren hergestellt werden. Je nach Wirtsorganismus können die erfindungsgemäßen Proteine (bei SAP - 2) glykosyliert oder, wenn sie in Prokaryonten synthetisiert werden, unglykosyliert sein.

Die Funktion der Toxizität gegen Mikroorganismen ist in verschiedenen Testsystemen zu ermitteln. In den Beispielen sind gängige Testverfahren beschrieben. (vgl. SELSTED et al. (1993) J. Biol. Chem., Vol. 268, p 6641 - 6648 und GANZ et al. (1985) J. Clin. Invest. Vol. 76, p 1427 - 1435)

Die Proteine der Erfindung wirken antibiotisch gegen Mikroorganismen, insbesondere gegen die Gram - negativen und Gram - positiven Familien, dabei bevorzugt gegen die Arten *E. coli* und *Staphylococcus aureus*.
Die Testsysteme sind ausführlich in Beispiel 3 beschreiben.

### VEKTOREN MIT DER ERFINDGSGEMÄßEN DNA

Ein weiterer Teil der Erfindung ist ein Vektor, der eine erfindungsgemäße cDNA oder DNA, weiterhin einen passenden Promotor und gegebenenfalls einen passenden Enhancer enthält. Ebenfalls kann auch noch eine Signal - Sequenz umfaßt sein. Vektoren sind ausführlich in den europäischen Publikationen EP 0 480 651, EP 0 462 632 und EP 0 173 177 beschrieben.

Eine weitere Ausführungsform der Erfindung besteht in einer eukaryontischen oder prokaryontischen Wirtszelle, die mit einem erfindungsgemäßen Vektor transformiert ist.

Die Erfindung umfaßt weiterhin ein Verfahren zum Herstellen eines erfindungsgemäßen Proteins unter Verwendung einer erfindungsgemäßen Wirtszelle, mit den Schritten:
Kultivieren der Wirtszelle,
Anreichern des Proteins, und
Reinigen des Proteins.

Weiterhin umfaßt die Erfindung ein Verfahren zum Synthetisieren von einem der erfindungsgemäßen Proteine, wobei nach der Festphasen - Methode oder nach der Flüssigphasen - Methode die Proteine synthetisiert werden.

Das Verfahren, bei dem die erfindungsgemäßen Protein hergestellt werden, hat die folgenden Stufen:
das Carboxyl - Ende einer zu koppelnden Aminosäure, deren Aminogruppen und gegebenenfalls funktionellen Gruppen der Seitenkette eine Schutzgruppe tragen, reagiert mit dem freien Amino - Ende der zu koppelnden Aminosäure oder des zu koppelnden Proteinfragments in Gegenwart eines Kondensationsreagenzes,
   und
   im Falle einer nicht - endständigen Aminosäure wird anschließend die α - Amino - Schutzgruppe der gekoppelten Aminosäure abgespalten und werden weitere Aminosäuren an die zu synthetisierende Protein - Kette nach den zuvor beschriebenen beiden Schritten gekoppelt oder
   im Falle einer endständigen Aminosäure wird gegebenenfalls anschließend die α - Amino - Schutzgruppe der gekoppelten Aminosäure abgespalten
   und
nach Kopplung der letzten Aminosäure im Falle der Festphasen - Methode wird das Protein von der Festphase abgespalten.

### ALLELISCHE MODIFIKATIONEN

Die meisten Deletionen, Insertionen und Substitutionen scheinen keine durchgreifende Änderung in der Charakteristik des Proteins der Erfindung zur Folge zu haben. Da es schwer ist, den genauen Effekt einer Substitution, einer Deletion oder einer Insertion im voraus anzugeben, muß die Funktion des veränderten Proteins mit der Funktion des erfindungsgemäßen Proteins verglichen werden. Die hierfür zu verwendenden Methoden sind in den Beispielen angegeben. Als Standard dient das Protein gemäß der SEQ ID NO: 1, ebenfalls auch das Protein, das nach Beispiel 1 gereinigt wird, und auch die Reinigungsmethode des Beispiels 1 für das Vergleichsprotein.

Der genetische Code ist degeneriert, das bedeutet, daß die meisten Aminosäuren von mehr als einem Codon aus drei Nukleotiden kodiert werden. Daher führen einige allelische Modifikationen auf der Ebene der Nukleotide nicht zu einer Änderung der Aminosäure - Sequenz. Daher ereignen sich allelische Modifikationen vornehmlich auf der Ebene der DNA und können sich sekundär auf die Aminosäure - Sequenz auswirken.

Die cDNA- oder DNA-Sequenzen, die die erfindungsgemäßen Proteine kodieren, können gemäß konventioneller Techniken modifiziert werden, um Varianten der erfindungsgemäßen Proteine herzustellen, die im wesentlichen die gleiche Aktivität wie die beschriebenen und charakterisierten Proteine der Erfindung besitzen. Dabei wird die Aktivität so gemessen, wie es in den Beispielen beschrieben ist. Eine derartige Homologie - Austestung wird in CUNNIGHAM et al. (1989) Science, Vol. 243, p 1330 und O'DOWD et al. (1988) J. Biol. Chem., Vol. 263, p 15985 beschrieben.

Aminosäuren können substituiert werden, wobei mit einem Protein- oder Peptid-Mapping die Aminosäuren in ihren Positionen substituiert werden können, wobei anschließend die Aktivität der Modifizierung gemessen wird. Hierbei sind experimentell ermittelbare Substituierungen möglich, die aufgrund der chemischen Struktur der Seitenketten nicht ohne weiteres voraussagbar ist.

Die Mutationen werden durch die Homologie (Similarity) zweier zum Vergleich anstehender Proteine definiert. Der Ausdruck Homologie umfaßt ähnliche Aminosäuren und Lücken in den Sequenzen der Aminosäuren (Homologie = similarity). Die erfindungsgemäßen Proteine haben Aminosäure - Sequenzen, die eine Homologie von wenigstens 80 %, bevorzugt 90 %, mehr bevorzugt 95 % und am meisten bevorzugt 98 % der erfindungsgemäßen Strukturen besitzt, wie sie durch die Sequenzen unter SEQ ID NO: 1 oder SEQ ID NO: 2 definiert sind und wie sie weiterhin nach der Reinigung gemäß der Beispiele erhalten werden.

Sequenzen von Proteinen lassen sich einfach verändern. Dabei werden die Aminosäuren in ihren jeweiligen Positionen ausgetauscht. Gleichzeitig ist notwendig, die so gewonnen Sequenzen auf ihre Funktion hin zu untersuchen. Der Aminosäure - Austausch kann nach zwei verschiedenen Methoden erfolgen.
Jede der Position eines Proteins wird nacheinander durch Alanin ersetzt. Anschließend wird die Funktion des jeweils um Alanin modifizierten Moleküls gemessen. Weicht der Meßwert von dem des Standard - Proteins ab, so ist diese Aminosäure an dieser Position des Proteins, an der nun ein Alanin angeordnet ist, für die Funktion essentiell. Hierdurch ergibt sich eine Karte des Proteins, aus der die konservativen Positionen und die einer Variation zugänglichen Positionen angegeben sind.
Eine andere Methode besteht darin, jede Position oder wesentliche Positionen eine Proteins gegen alle 20 natürlichen Aminosäuren auszutauschen. Anschließend wird von all diesen Modifikationen die Funktion ausgetestet. Die Methode wird beschrieben in Ronald FRANK (1992) Spot - Synthesis: easy technique for the poitionally addressable, parallel chemical synthesis on a membrane support. Tetrahedron Vol . 48, No 42, pp 9217 - 9232.
Beide Methoden sind besonders für Peptide geeignet, da diese mit der Festphasensynthese hergestellt werden. Dennoch läßt sich diese Methode mit Leichtigkeit auch für den Fachmann auf Proteine übertragen, wobei die Proteine in Zellen synthetisiert werden. Hierbei ist die Veränderung der DNA wesentlich, die jedoch bei den heutigen Techniken gezielt erfolgen kann.

Das Verfahren zur Modifizierung der Aminosäure - Sequenz kann wie folgt ausgeführt werden:
(a) Mindestens eine Aminosäure in der Sequenz des Proteins wird durch eine natürliche oder auch gegebenenfalls durch eine nicht natürliche Aminosäure ersetzt.
(b) Das modifizierte Protein wird nach jeder Substituierung auf die antibiotische Funktion gegenüber Mikroorganismen ausgetestet und die am meisten antibiotischen Proteine werden selektioniert.
(c) In einem weiteren Schritt werden die am meisten antibiotischen Proteine mindestens in einem weiteren Zykius gemäß der Punkte (a) und (b) durchlaufen.

Das Resultat einer solchen Modifizierung kann ein Protein sein, welches mit der ursprünglichen Sequenz nur noch Teile gemeinsam hat.

Wie zuvor erwähnt, umfaßt die Erfindung auch Modifikationen der DNA oder cDNA. Diese modifizierten Sequenzen hybridisieren unter stringenten Bedingungen mit den DNA-Sequenzen, die die erfindungsgemäßen Proteine kodieren (siehe Sequenzen unter aa); cc) und ee)). Die cDNA- oder DNA-Sequenzen haben Nukleotid - Sequenzen, die eine Identität einschließlich kurzer (bis 15 Nukleotide) Deletionen und Insertionen von wenigstens 70 %, bevorzugt 82 %, mehr bevorzugt 90 % und am meisten bevorzugt von 95 % mit den erfindungsgemäßen cDNA- oder DNA-Sequenzen besitzen (siehe aa), cc) und ee)). Die Identität einschließlich der kurzen (bis 15 Nukleotide) Deletionen und Insertionen kann durch eine Hybridisierung gemessen werden, wie sie in R. KNIPPERS, Molekulare Genetik, 1982, dritte Auflage, Georg Thieme Verlag Stuttgart, New York beschrieben ist. Außerdem sind Standard - Rechenprogramme dem Fachmann bekannt, mit deren Hilfe Homologie zu berechnen ist.

Die Erfindung umfaßt weiterhin eine cDNA oder DNA mit mindestens einer der Sequenzen SEQ ID NO: 3 oder 4
oder
Nukleotidsequenzen, die mit der SEQ ID NO: 3 oder 4 unter selektiven, stringenten Bedingungen hybridisiert.
Stringente Bedingungen liegen dann vor, wenn die Salze, deren Konzentration, die Temperatur die anorganischen und organischen Lösungsmittel in typischer Form kontrolliert werden, wie dieses bei der etablierten Hybridisierungstechnik praktiziert wird. Stringente Temperatur - Bedingungen schließen Temperaturen von mindestens 30 °C, bevorzugt mindestens 37 °C, mehr bevorzugt mindestens 45 °C, noch mehr bevorzugt mindestens 55 °C, noch vorteilhafter mindestens 65 °C und am meisten bevorzugt mindestens 70 °C ein. Stringente Salzkonzentration umfassen weniger als 1000 mM, bevorzugt weniger als 700 mM, mehr bevorzugt weniger als 400 mM, noch mehr bevorzugt weniger als 300 mM, vorteilhafter weniger als 200 mM und am meisten bevorzugt 150 mM. Die Kombination der Parameter ist wichtiger als der Bezug auf einen einzelnen Parameter. (WETMUR et al. (1968) J. Mol. Biol., Vol. 31, p 349)

### POSTTRANSLATIONALE MODIFIKATIONEN

Dem Fachmann ist bekannt, dass die Proteine der vorliegenden Erfindung auch posttranslationale Modifikationen ihrer Sequenz aufweisen können, sofern die antimikrobielle und / oder antibiotische Aktivität bei den posttranslational modifizierten Proteinen erhalten bleibt.

Unter posttranslationalen Modifikationen versteht man Veränderungen, die während oder nach der Translation auftreten. Hierzu zählen die Glykosylierung, die Ausbildung von Disulfid - Brücken, die chemische Modifikationen der Aminosäuren, so zum Beispiel die Sulfatierung, die im Zusammenhang mit dem Hirudin beschrieben ist. (J.W. FENTON (1989) "Thrombin Interactions with Hirudin", Seminars in Thrombosis and Hemostasis Vol. **15, p** 265 - 268)
Die Glykosylierung ist eine wesentliche Funktion des endoplasmatischen Retikulums und/oder des Golgi-Apparates. Die Sequenz und die Verästelung der Oligosaccharide wird in dem endoplasmatischem Retikulum gebildet und in dem Golgi-Apparat verändert. Die Oligosaccharide können N-verknüpfte Oligosaccharide (Asparagin-verknüpfte) oder O-verknüpfte Oligosaccharide (Serin-, Threonin- oder Hydroxylysin-verknüpfte) sein. Die Form der Glykosylierung ist von dem produzierenden Zelltyp und von der Art abhängig, von der der entsprechende Zelltyp stammt. Das Ausmaß und die Art der Glykosylierung kann durch Substanzen beeinflußt werden, wie es in der europäischen Publikation EP 0 222 313 beschrieben ist. Die Variation der Glykosylierung kann die Funktion des Proteins verändern.
Proteine bilden häufig kovalente Bindungen innerhalb der Ketten aus. Diese Disulfid - Brücken werden zwischen zwei Cysteinen hergestellt. Dabei wird das Protein spezifisch gefaltet. Die Disulfid - Brücken stabilisieren die dreidimensionale Struktur der Proteine.

### ISOLIERUNG UND HERSTELLUNG DER ERFINDUNGSGEMÄßEN PROTEINE

Die Erfindung umfaßt weiterhin ein Verfahren zur Reinigung von erfindungsgemäßen Proteinen, wobei das Verfahren aus folgenden Schritten besteht:
(i) Extrahieren der Proteine aus natürlichen menschlichen Epithel - Zellen, transfizierten Zellen oder Hautschuppen oder Zellkulturen, die gegenüber Mikroorganismen gegebenenfalls exponiert wurden,
(ii) Auftragen des Extraktes auf eine Affinitätssäule mit anschließender Reversed Phase HPLC und
   Eluieren über einen Salzgradienten, mit Säuren oder organischen Eluenten,
   oder
(iii) Auftragen des Extraktes auf eine HPLC - Säule und Eluieren mit Salzen.

Die Reinigung ist ausführlich in den Beispielen beschrieben.
Bevorzugt ist eine Mikro - Mono S -HPLC - Säule.
Die Proteine werden bevorzugt gemäß Beispiel 1 gereinigt. Jedoch sind auch andere Isolierungs - und Reinigungsmethoden möglich:
Methods of Enzymology, Volume **182**: Guide to Protein Purification, ed.
Murray P. DEUTSCHER, Academic Press, 1990;
Protein Purification Application - A Practical Approach, ed. E.L.V. HARRIS and S. ANGEL, IRL-Press 1990;
Protein Purification, Principles and Practice, Ropert SCOPES, Springer-Verlag 1982; and
Protein Purification, Principles, High Resolution Methods and Applications, ed. H.-C. JANSON and L. RYDEN, VCH publishers 1989.

### VERWENDUNG ALS ARZNEIMITTEL

Die Proteine gemäß der Erfindung besitzen pharmakologische Effekte und sind deshalb als pharmazeutische Wirkstoffe verwendbar. Die Erfindung umfaßt ebenfalls ein Arzneimittel, das eines der erfindungsgemäßen Proteine oder ein Gemisch davon enthält. Weiterhin gehört zur Erfindung eine pharmazeutische Zusammensetzung, die eines der erfindungsgemäßen Proteine oder ein Gemisch erfindungsgemäßer Proteine enthält, in Gegenwart von pharmazeutisch verträglichen und annehmbaren Verbindungen und Trägem. Ebenfalls umfaßt die Erfindung eine pharmazeutische Zusammensetzung, die eines der pharmazeutisch aktiven, erfindungsgemäßen Proteine oder deren Gemisch und ein pharmazeutisch verträgliches Salz oder einen pharmazeutisch verträglichen Träger enthält.

Insbesondere zeigen die erfindungsgemäßen Proteine gemäß der SEQ ID NO: 1 eine toxische oder antibiotische Wirkung gegenüber Mikroorganismen, insbesondere gegenüber den Gruppen der Gram - negativen und Gram - positiven Bakterien, bevorzugt bei den Arten *E. coli* und *St. aureus*. Testverfahren sind in dem Beispiel 2 beschrieben.

Die Versuchsergebnisse dieser *in vitro* Tests zeigen, daß die erfindungsgemäßen Proteine als Arzneimittel oder zur medizinischen Behandlung verwendet werden können. Diese Versuchsergebnisse lassen sich von den *in vitro* Testsystemen auf ein *in vivo* System übertragen, da es sich bei den Tests um etablierte Versuchsanordnungen handelt. Die Proteine der Erfindung können deshalb zur Behandlung und Prävention von Infektionen durch Mikroorganismen dienen. Die Proteine der Erfindung können als ein antibiotisches Medikament bei Säugern, insbesondere Menschen, zur Behandlung von Infektionen und / oder zur Infektionsprophylaxe eingesetzt werden

Die Erfindung liefert weiterhin
(i) die Verwendung eines der erfindungsgemäßen Proteine oder deren Gemisch zur Herstellung eines Medikaments zur Behandlung von
   Infektionen, die von Mikroorganismen verursacht wurden oder zur Prävention solcher Infektionen;
(ii) ein Verfahren zur Behandlung von
   Infektionen, die von Mikroorganismen verursacht wurden oder zur Prävention solcher Infektionen,
   welches Verfahren eine Verabreichung einer Proteinmenge gemäß der Erfindung umfaßt, wobei die Menge die Krankheit unterdrückt, und wobei die Proteinmenge einem Patienten gegeben wird, der ein solches Medikament benötigt;
(iii) eine pharmazeutische Zusammensetzung zur Behandlung von Infektionen, die von Mikroorganismen verursacht wurden oder zur Prävention solcher Infektionen,
   welche Behandlung eines der erfindungsgemäßen Proteine oder deren Gemisch und wenigstens einen pharmazeutisch verträglichen Träger und Zusatz umfaßt.

Für diese therapeutische Wirkung sind unterschiedliche Dosen geeignet. Sie hängen beispielsweise von dem verwendeten Protein, von dem Wirt, von der Art der Verabreichung und von der Art und der Schwere der zu behandelnden Zustände ab. Im allgemeinen sind jedoch bei Tieren zufriedenstellende Resultate zu erwarten, wenn die tägliche Dosen einen Bereich von 2 µg bis 2000 µg pro kg Körpergewicht umfassen. Bei größeren Säugetieren, beispielsweise dem Menschen, liegt eine empfohlenen tägliche Dosis im Bereich von 2 bis 2000 µg pro kg Körpergewicht, wenn das nach Beispiel 1 oder 2 gereinigte Protein verwendet wird. Zum Beispiel wird diese Dosis zweckmäßiger Weise in Teildosen bis zu viermal täglich verabreicht. Die täglich Dosis bei Prävention beträgt ein zehntel der Menge, die bei einer Infektion eingesetzt wird.
Das erfindungsgemäße Protein wird bevorzugt lokal oder epithelial verabreicht, so auch in den oberen und unteren Luftwegen.

Die erfindungsgemäßen Proteine können auf jedem üblichen Weg verabreicht werden, auch in Form von Cremen, Gele, halbfeste Arzneiformen, Suspensionen oder Inhalationslösungen oder Inhalationspulver.

Die vorliegende Erfindung stellt pharmazeutische Zusammensetzungen zur Verfügung, die eines der erfindungsgemäßen Proteine oder deren Gemisch und wenigstens einen pharmazeutisch verträglichen Träger oder Zusatz umfassen. Solche Zusammensetzungen können nach bekannten Verfahren hergestellt werden. Dabei ist auf Remington's Pharmaceutical Science, 15^{th} ed Mack Publishing Company, East Pennsylvania (1980) hinzuweisen.

Weiterhin sind mit der erfindungsgemäßen DNA oder cDNA transfizierte syngene oder allogene humane Zellen als Medikament einsetzbar, indem diese Zellen auf dem Epithelgewebe aufgetragen werden oder sich in der Matrix eines Wundverbandes befinden.

### DEFINITIONEN:

,.Antimikrobiell" bedeutet, daß die erfindungsgemäßen Proteine
(i) das Wachstum und / oder die Proliferation von Mikroorganismen inhibieren und / oder verhindern und / oder
(ii) die Mikroorganismen oder deren Strukturen zerstören.

"Antibiotisch" bedeutet, daß die erfindungsgemäßen Proteine nachteilig auf die normalen biologischen Funktion der Mikroorganismen einwirken, wobei dieses Tod oder Zerstörung, ebenso auch Verhinderung von Wachstum oder Proliferation der Mikroorganismen, weiterhin auch Beeinträchtigung der Stoffwechselfunktionen bedeutet. Antibiotisch umfaßt somit den Begriff antimikrobiell. Eine antibiotische Wirkung kann auch bei Viren vorliegen. Daher umfaßt antibiotisch auch antiviral.

"Antiviral" bedeutet, daß DNA - und RNA - Viren mit Hilfe der Proteine der Erfindung bekämpft werden können. Hierbei sind verschiedene Eingriffsmöglichkeiten sinnvoll. Die Viren in ihrer Ruheform können beeinflußt werden. Die Anhaftphase an oder das Eindringen in den Wirt kann gestört werden, der Aufenthalt oder die Vermehrung (temperente oder virulente Phase) in dem Wirt kann beeinträchtigt werden.

Die erfindungsgemäßen Proteine können auch zur Wundheilung eingesetzt werden.

"Wundheilung" bedeutet, daß zum Beispiel die Kontraktion von Wunden beschleunigt wird, daß Bindegewebe im Wundbereich angesiedelt wird, daß Collagen angelagert wird. Ebenso sind Brandverletzung gut mit den Proteinen der Erfindung zu behandeln. Dabei kann ein Wundverband mit Proteinen angereichert sein oder Proteine von speziellen, insbesondere transfizierten Zellen im Wundverband exprimiert werden.

"Mikroorganismen" umfassen die Prokaryonten mit Eubakterien und Archaebakterien, die Pilze (Mycota mit Myxomyceten, Phycomyceten, und Eumyceten), die pflanzlichen und tierischen Einzeller, und Viren.

Der Ausdruck "Protein" umfaßt alle Längen an Aminosäuresequenzen, somit auch Peptide. Dabei können sich die Proteine auch aus verschiedenen Ketten zusammensetzen, die durch kovalente Bindungen oder Van der Vaal sche Kräfte verbunden sind.

### KOMBINATION MIT ANTIBIOTIKA:

Die erfindungsgemäßen Proteine können zusammen mit Antibiotika zum Beispiel aus der folgenden Gruppe verabreicht werden:
Bacitracin, Gramicidin, Polymyxin, Vancomycin, Teichoplanin, aminoglycoside, Penicillin, Monobactam.

### Diagnostikum:

Die Erfindung umfaßt weiterhin die Verwendung von mindestens einem erfindungsgemäßen Protein zur Herstellung von monoklonalen Antikörpern oder Fragmenten davon.

Ebenfalls umfaßt die Erfindung die Verwendung von einem erfindungsgemäßen Antikörper oder Fragment davon als Diagnostikum.
Dabei sollen die erfindungsgemäßen Protein in Körpergeweben und Körperflüssigkeiten nachgewiesen werden. Auch können damit Nachweisverfahren durch Kopplung von Liganden an die erfindungsgemäßen Proteine hergestellt werden.

### BEISPIELE

### Beispiel 1

### Gewinnung von SAP-2

### 1.1. Isolierung:

50 g läsionaler Psoriasisschuppen wurde unter sauren Bedingungen bei Anwesenheit von Ethanol extrahiert und eingeengt. Dabei wurde dem Verfahren gefolgt, das beschrieben ist in J.M. SCHRÖDER, (1997) Methods in Enzymology, Vol 288. p 266 - 296.
Nach Diafiltrieren gegen 0,02 mol/l Natriumphosphatpuffer, pH 8 und Zentrifugation wurde der Überstand an einer Bakterien - Affinitätssäule (*E. coli* oder *Staph. aureus*), die durch Kopplung hitze - inaktivierter (70 °C über eine Stunde) *E. coli* oder *Staphylococcus aureus -* Bakterien an N-hydroxysuccinimid - aktivierte Sepharose - Säule (Pharmacia) (10 x 5 mm) hergestellt worden war, chromatographiert.
Dazu wurde die Säule zunächst mit dem Äquilibrierungspuffer gewaschen und anschließend gebundene Proteine mit einem sauren Puffer (0,1 mol/l Glycin - Puffer, pH 3 mit 1 mol/l NaCl) eluiert.
Das gebundene Protein enthaltende Eluat wurde gegen 0,1 % wäßriger Trifluoressigsäure ― Lösung diafiltriert und analog zur Isolierung von chemotaktischen Peptiden (vgl. J.M. SCHRÖDER, (1997) Methods in Enzymology, Vol 288. p 266 - 296) zunächst einer präparativen Reversed Phase HPLC - Trennung unterzogen. 20µl der jeweiligen Fraktionen wurden lyophilisiert, in 5 µl einer 0,01 prozentigen wäßrigen Essigsäure - Lösung aufgenommen und mit Hilfe eines Plattendiffusions - Testsystems (siehe Beispiel 3) (zur Identifikation antimikrobieller oder antibiotischer Proteine) analog zu M. E. SELSTED et al. (1993) J. Biol. Chem., Vol. 268, p 6641 - 6648 hinsichtlich der Anwesenheit antimikrobieller Peptide (mit *Staph. aureus* und *E. coli* als Test - Bakterium) analysiert.

Bei 40 % Acetonitril eluierte, antimikrobiell aktive Proteine wurden anschließend
- analog zur Isolierung chemotaktischer Peptide (J.M. SCHRÖDER, (1997) Methods in Enzymology, Vol 288. p 266 - 296) - einer Mikro - Mono S - HPLC
- Trennung mit Hilfe des Smart - HPLC - System unterzogen.
SAP-2 wurde bei 0,8 mol/l NaCl eluiert.
Eine anschließende Mikro - Reversed Phase - HPLC - Analyse mit Hilfe einer C - 18 RP - Säule ergab einen bei 52 % Acetonitril eluierenden Proteinpeak, der nach SDS - Gel - Elektrophorese (durchgeführt nach der Methode gemäß J.M. SCHRÖDER, (1997) Methods in Enzymology, Vol 288. p 266 - 296 eine einzelne Proteinbande oder zwei Banden entsprechend der Mobilität von zirka 20 kDa ergab.

### 1.2. Sequenzierung von Fragmenten

Sequenzierungsversuche ergaben die aminoterminale Sequenz mit der Numerierung aus der vollständigen Sequenz.

Pro Lys Gly Met Thr Ser Ser Gln Trp Phe Lys Ile Gln His Met
            5                   10                  15

Gln Pro Ser Pro Gln Ala Cys Asn Ser Ala Met Lys Asn Ile Asn
            20                  25                  30

Lys His Thr Lys Arg Cys Lys Asp
            35

Der Edman Abbau des Peptidfragments lieferte eine Sequenz , die dem C –
Terminus entspricht:

Asp Ser Gln Gln Phe His Leu Val Pro Val
        115                 120
His Leu Asp Arg Val Leu
        125

### 1.3. Biologische Aktivität von SAP-2: MIC

Antimikrobielle Aktivität gegen *Staph aureus:* < 100 µg/ml
gegen *E. coli*: < 50 µg/ml

RNase - Aktivität: 1.2 µg SAP - 2 verdaute in einer Stunde bei 37 °C zirka 5 µg humane RNA.

### Beispiel 2

### 2. Bestimmung antimikrobieller Aktivität

### 2.1. Kultivierung der Mikroorganismen

Folgende Mikroorganismen wurden für die Versuche verwendet:
- *Escherichia coli (E. coli) -* ATCC (American Type Culture Collection ) Nr. 11303
- *Pseudomonas aeruginosa -* ATCC Nr. 15442
- *Staphylococcus aureus* (Klinische Isolate der Hautklinik ― Kiel)
- *Staphylococcus epidermidis* (Klinische Isolate der Hautklinik - Kiel)
- *Candida albicans* (Klinische Isolate der Hautklinik - Kiel)

Die Mikroorganismen wurden auf Trypticase-Soy-Broth (TSB) - Agarplatten bei 37°C kultiviert. Wurden sie längere Zeit nicht benötigt, lagerten sie bei 4°C.
Für die Versuche wurde jeweils eine Einzelkolonie der entsprechenden Mikroorganismen in 40 ml TSB-Medium angeimpft und über Nacht bei 37°C unter Schütteln (250 Upm) inkubiert. Zur Quantifizierung der Mikroorganismen wurde die optische Dichte der Übemachtkulturen bei 620 nm (OD₆₂₀) gemessen und die Koloniezahl durch Ausplattieren von entsprechenden Verdünnungsstufen bestimmt

### 2.2 Plattendiffusions-Test

Um möglichst schnell und sensitiv Fraktionen der einzelnen chromatographischen Reinigungsschritte (vgl. 3.3.) auf antimikrobielle Aktivität zu untersuchen, wurde ein radialer Plattendiffusions-Test (vgl. Hiemstra et al., 1993) verwendet.
Um Bakterien aus einer logarithmischen Wachstumsphase zu erhalten, wurden 20 µl einer 40 ml Übernachtkultur von *E. coli* oder *Staphyloccccus aureus in* 8 ml Trypticase - Soy - Broth (TSB) geimpft und 3,5 h bei 37°C inkubiert. Die Bakterien wurden dann 10 min bei 1000 g abzentrifugiert, mit 4°C kalten Natrium-Phosphat-Puffer (10 mM. pH = 7,4) gewaschen, in 1 ml Natrium-Phosphat-Puffer resuspendiert und durch Bestimmung der OD₆₂₀ Quantifiziert.
Etwa 1x10⁶ Bakterien wurden dann in 8 ml vorgewärmtes (42 °C) Agarose - Medium gegeben, welches sich aus 1 % Agarose in Natrium-Phosphat-Puffer + 1 % TSB-Medium (v/v) + 0,03% Tween 80 (v/v) zusammensetzte. Nach Einfüllen dieses mit Bakterien versetzten Agarose-Mediums in eine Petrischale (⌀ = 10cm ;Sarstedt, Newton) und anschließender Abkühlung bei Raumtemperatur, wurden in die nun verfestigte Agaroseschicht Löcher von 3 mm Durchmesser gestanzt. In diese Löcher wurden dann 5 µl der zu testenden Substanz in 0,01% Essigsäure gegeben.
Nach Beendigung der Inkubationszeit wurde die Agaroseschicht mit 42°C warmen 2 x TSB-Medium + 1 % Agarose überschichtet und bei 37°C inkubiert. Nach ca. 20 bis 24 Stunden konnte man die Hemmhöfe der antimikrobiellen oder antibiotischen Fraktionen im Bakterienrasen deutlich erkennen. Die relativen antimikrobiellen oder antibiotischen Aktivitäten wurden durch den jeweiligen Durchmesser der Hemmhöfe bestimmt.

### 2.3. Flüssigkultur-Test

Um den dosisabhängigen Wirkungsbereich eines antimikrobiellen oder antibiotischen Proteins abschätzen zu können, wurde ein Flüssigkultur-Testsystem verwendet ( vgl. Ganz et al., 1985).

Etwa 10 µl einer 1x10⁷/ml-Verdünnung der entsprechenden Mikroorganismen in Natrium-Phosphat-Puffer (vgl. 3.1.) wurden zu 80 µl Natrium-Phosphat-Puffer zusammen mit 1,25% TSB - Medium (v/v) gegeben. Hinzugefügt wurden 10 µl 0,01% Essigsäure mit den entsprechenden Konzentrationen des antimikrobiellen oder antibiotischen Proteins (100 µg/ml, 50 µg/ml, 25 µg/ml, 12,5 µg/ml, und 6,25 µg/ml).
Diese Ansätze wurden in einer 96-Loch-Platte (Becton Dickinson, Heidelberg) 3 h bei 37°C unter leichtem Schütteln (150 Upm) inkubiert. Nach der Inkubationszeit wurden von je 50 µl der Ansätze zehnfache Verdünnungsreihen mit Natrium-Phosphat-Puffer erstellt und jeweils in 3 Parallelen auf TSB-Agarplatten ausplattiert (100 µl). Nach 24 bis 36 Stunden Wachstum wurden die Kolonien ausgezählt.
Als Kontrolle wurde je ein Ansatz nur mit 10 µl 0,01% Essigsäure (ohne Protein) einmal direkt vor der Inkubation und einmal nach 2 h Inkubation bei 37°C ausplattiert.

### 3. Versuchsergebnisse

SAP-2 wurde in den angegebenen Konzentrationen bei 37° C für 3 Stunden mit 1 • 10⁵ KBE / ml der jeweiligen Mikroorganismen in 100 µl 10 mM Natriumphosphat - Puffer (pH = 7,4) zusammen mit 1 % TSB inkubiert. Die antimikrobielle Aktivität von SAP-2 wurde durch Auszählung der KBE am nachfolgenden Tag bestimmt. Zuvor wurden 100 µl der jeweiligen Ansätze in zehnfachen Verdünnungsstufen) auf TSB - Platten ausplattiert. Danach wurden die Platten bei 37° C über Nacht inkubiert.
Es ergibt sich eine LD₉₀ von 4 - 7,5 µg/ml für Propionibacterium acnes; 7,5 - 15 µg/ml für Staphylococcus aureus und Pseudomonas aeruginosa; weiterhin 15 - 30 µg/ml für Candida albicans.

### Beispiel 5

### 5. Biochemische Charakterisierung antimikrobieller oder antibiotischer Proteine mit SDS-Polyacrylamid-Gelelektrophorese (SDS-PAGE)

Zur Bestimmung der relativen Molekülmasse wurde die Tricine-SDS-Polyacrylamid-Gelelektrophorese verwendet (Schägger und Jagow, 1987), weiche es gestattet, kleine Proteine unter 10 kDa sehr effizient aufzutrennen.

Die Durchführung geschah nach dem Protokoll der Autoren (Schägger und Jagow, 1987) in einer vertikalen Gelelektrophoresekammer, wobei ein 16,5% Polyacrylamidgel mit einem Anteil von 6% Bisacrylamid und 6 M Harnstoff verwendet wurde.

Die Proben wurden vor dem Auftrag durch 0, 1 M DTT und Aufkochen denaturiert. Als molekularer Größenmarker diente der Standard S-17 (Sigma, St.Louis, USA). Nach dem Elektrophoreselauf wurde das Gel einer Silberfärbung unterzogen:
- Zuerst wurde das Gel 30 min fixiert (30% Ethanol. 10% Eisessig).
- Anschließend erfolgte eine 30 min Inkubation in "Farmer's reducer"-Lösung.
- Dann wurde das Gel 3 x 10 min mit H₂O gewaschen und 20 min in Silbernitratlösung gefärbt.
- Abschließend erfolgte eine 10-15 min Inkubation in Entwickler-Lösung.
- Die Entwicklung wurde durch 5% Essigsäure abgestoppt und das Gel anschließend photographiert.

Für eine schnelle Analyse der HPLC-Fraktionen wurde das SDS-Page-Phast-System (Pharmacia, Freiburg) mit fertigen High-Density-Gelen (Pharmacia) nach Angaben des Herstellers benutzt. Als Größenmarker diente der S-17-Marker von Sigma (s.o.). Die Detektion der aufgetrennten Moleküle erfolgte mit der oben beschriebenen Silberfärbung.

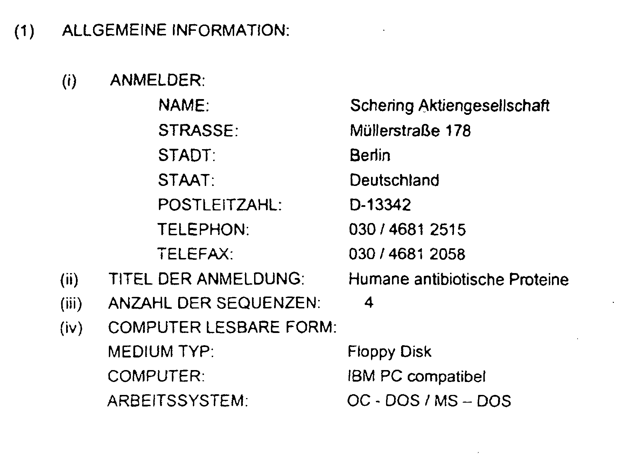

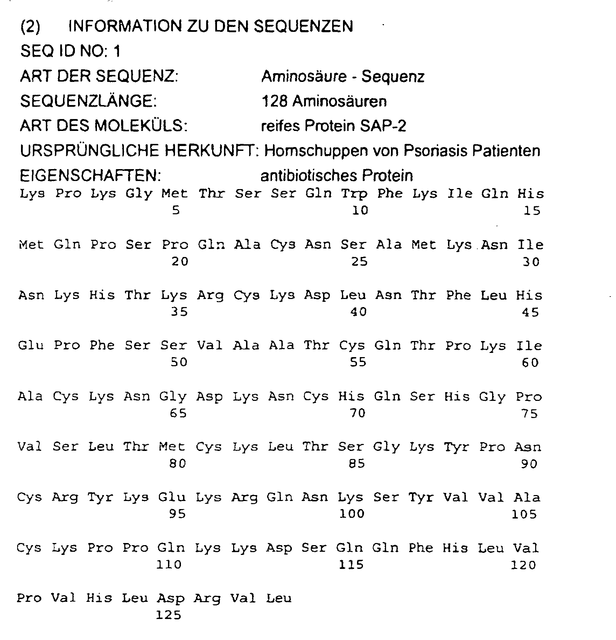

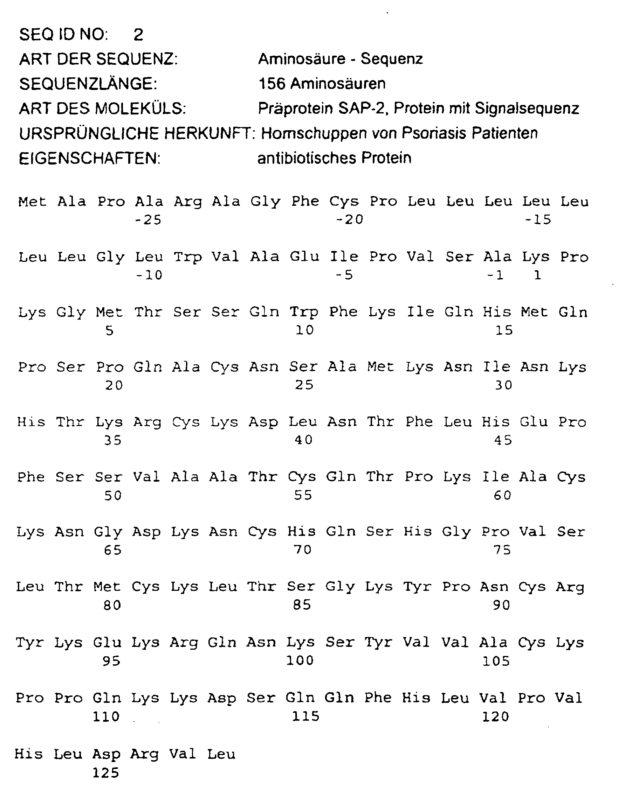

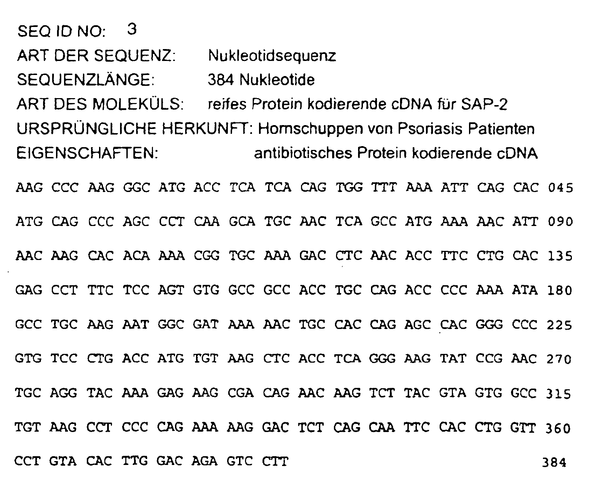

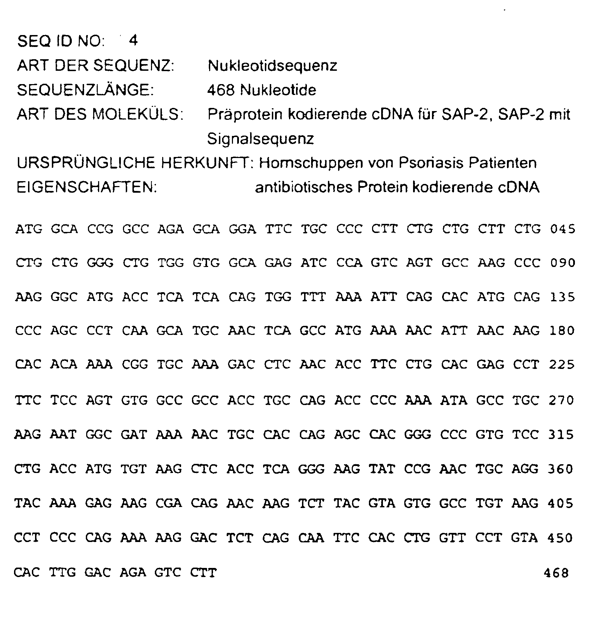

## Patentansprüche

1. Protein, das als aktives, reifes Protein / Peptid (Protein) folgende Sequenz aufweist:
a) SEQ ID NO: 1 (SAP-2)
oder
b) das als aktives, reifes Protein allelische Modifikationen der zuvor unter a) genannten Aminosäure-Sequenz aufweist, wobei bis zu 30 Aminosäuren der Aminosäure-Sequenz substituiert, deletiert oder insertiert sind,
wobei die antimikrobielle und / oder antibiotische Aktivität des unter a) definierten aktiven, reifen Proteins erhalten bleibt.

2. Protein, das eine Signalsequenz und ein reifes Protein nach Anspruch 1 umfasst, wobei das Protein folgende Sequenz aufweist:
a) SEQ ID NO: 2 (PreSAP-2)
oder
b) wobei das Protein allelische Modifikationen der zuvor unter a) genannten Aminosäure-Sequenz aufweist, wobei bis zu 35 Aminosäuren der Aminosäure-Sequenz substituiert, deletiert oder insertiert sind,
wobei die antimikrobielle und / oder antibiotische Aktivität des unter a) definierten aktiven, reifen Proteins erhalten bleibt.

3. Protein nach einem der vorherigen Ansprüche, wobei an dem N-Terminus und / oder C-Terminus Schutzgruppen angeordnet sind.

4. Protein nach einem der vorherigen Ansprüche, welches ein rekombinantes Protein ist.

5. cDNA oder DNA,
wobei die cDNA oder DNA folgende Aminosäure-Sequenz kodiert:
aa) SEQ ID NO: 1 (SAP-2); oder
SEQ ID NO: 2 (PreSAP-2);
oder
bb) wobei die cDNA oder DNA allelische Modifikationen einer der Aminosäure-Sequenzen unter aa) kodiert, worin bis zu 30 (SAP-2) bzw. 35 (PreSAP-2) Aminosäuren der Aminosäure-Sequenz substituiert, deletiert oder insertiert sind, wobei die antimikrobielle und / oder antibiotische Aktivität des aktiven, reifen Proteins erhalten bleibt.

6. cDNA oder DNA nach Anspruch 5, wobei die cDNA oder DNA ein reifes Protein kodiert.

7. cDNA nach Anspruch 5 oder 6,
bestehend aus einer der folgenden Nukleotidsequenzen:
aa) SEQ ID NO: 3; (cDNA-SAP-2); oder
SEQ ID NO: 4; (cDNA-PreSAP-2)
oder
bb) wobei die cDNA oder DNA eine allelische Modifikation einer der Nukleotidsequenzen unter aa) aufweist, wobei bis zu 90 Nukleotide substituiert, deletiert oder insertiert sind, wobei die antimikrobielle und / oder antibiotische Aktivität des Proteins, das von der allelischen Modifikation einer der Nukleotidsequenzen unter aa) kodiert wird, erhalten bleibt.

8. Vektor, der eine cDNA oder DNA nach einem der Ansprüche 5 bis 7, weiterhin einen Promotor und gegebenenfalls einen Enhancer enthält.

9. Eukaryontische oder prokaryontische Wirtszelle, die mit dem Vektor nach Anspruch 8 transformiert ist.

10. Protein nach einem der Ansprüche 1 bis 4 zur Verwendung als pharmazeutischer Wirkstoff.

11. Pharmazeutische Zusammensetzung, die eines der Proteine nach einem der Ansprüche 1 bis 4 oder ein Gemisch davon enthält, in Gegenwart von pharmazeutisch verträglichen und annehmbaren Verbindungen und Trägern.

12. Verwendung eines der Proteine nach einem der Ansprüche 1 bis 4 oder deren Gemisch zur Herstellung eines Medikaments zur Behandlung von Infektionen, die von Mikroorganismen verursacht wurden oder zur Prävention solcher Infektionen.

13. Verfahren zum Synthetisieren von einem der Proteine nach einem der vorherigen Ansprüche 1 bis 4, wobei die Proteine nach der Festphasen-Methode oder nach der Flüssigphasen-Methode synthetisiert werden.

14. Verfahren zur Herstellung von monoklonalen Antikörpern oder Antikörper-Fragmenten unter Verwendung von mindestens einem Protein nach einem der vorherigen Ansprüche 1 bis 4.

15. Antikörper oder Fragmente der Antikörper, die Domänen auf dem reifen Protein nach einem der vorherigen Ansprüche 1 bis 4 spezifisch erkennen.

16. Antikörper oder Fragmente der Antikörper die Domänen auf dem reifen Protein nach einem der Ansprüche 1 bis 4 spezifisch erkennen, zur Verwendung als Diagnostikum.

17. Wundverband
mit mindestens einem Protein nach einem der vorherigen Ansprüche 1 bis 4,
oder
mit syngenen oder allogenen humanen Zellen, die mit cDNA nach einem der Ansprüche 5-7 transfiziert sind.

## Claims

1. Protein, which, as an active mature protein/peptide (protein), has the following sequence:
a) SEQ ID NO:1 (SAP-2)
or
b) which, as an active mature protein, has allelic modifications of the amino acid sequence mentioned above under a), with up to 30 amino acids of the amino acid sequence having been substituted, deleted or inserted,
with the antimicrobial and/or antibiotic activity of the active mature protein defined under a) being retained.

2. Protein, which comprises a signal sequence and a mature protein according to claim 1 and which has the following sequence:
a) SEQ ID NO:2 (PreSAP-2)
or
b) which protein has allelic modifications of the amino acid sequence mentioned above under a), with up to 35 amino acids of the amino acid sequence having been substituted, deleted or inserted,
with the antimicrobial and/or antibiotic activity of the active mature protein defined under a) being retained.

3. Protein according to any of the preceding claims, in which protective groups are present on the N terminus and/or the C terminus.

4. Protein according to any of the preceding claims, which is a recombinant protein.

5. cDNA or DNA,
which encodes the following amino acid sequence:
aa) SEQ ID NO:1 (SAP-2); or
SEQ ID NO:2 (PreSAP-2);
or
bb) which encodes allelic modifications of either of the amino acid sequences under aa), in which sequences up to 30 (SAP-2) or 35 (PreSAP-2) amino acids of the amino acid sequence have been substituted, deleted or inserted, with the antimicrobial and/or antibiotic activity of the active mature protein being retained.

6. cDNA or DNA according to claim 5, which encodes a mature protein.

7. cDNA according to claim 5 or 6,
consisting of any of the following nucleotide sequences:
aa) SEQ ID NO:3; (cDNA-SAP-2); or
SEQ ID NO:4; (cDNA-PreSAP-2)
or
bb) which has an allelic modification of either of the nucleotide sequences under aa), with up to 90 nucleotides having been substituted, deleted or inserted, with the antimicrobial and/or antibiotic activity of the protein encoded by the allelic modification of either of the nucleotide sequences under aa) being retained.

8. Vector, comprising a cDNA or DNA according to any of claims 5 to 7, furthermore a promoter and, where appropriate, an enhancer.

9. Eukaryotic or prokaryotic host cell, transformed with the vector according to claim 8.

10. Protein according to any of claims 1 to 4 for use as a pharmaceutical.

11. Pharmaceutical composition, comprising any one of the proteins according to any of claims 1 to 4 or a mixture thereof, in the presence of pharmaceutically suitable and acceptable compounds and carriers.

12. Use of any one of the proteins according to any of claims 1 to 4 or a mixture thereof for preparing a medicament for the treatment of infections caused by microorganisms or for the prevention of such infections.

13. Process for synthesizing any of the proteins according to any of the preceding claims 1 to 4, in which the proteins are synthesized according to the solid-phase method or the liquid-phase method.

14. Process for preparing monoclonal antibodies or antibody fragments by using at least one protein according to any one of claims 1 to 4.

15. Antibodies or fragments of the antibodies, which specifically recognize domains on the mature protein according to any one of claims 1 to 4.

16. Antibodies or fragments of the antibodies, which specifically recognize domains on the mature protein according to any of claims 1 to 4, for use as a diagnostic agent.

17. Wound dressing,
containing at least one protein according to any one of claims 1 to 4,
or
containing syngeneic or allogeneic human cells transfected with cDNA according to any one of claims 5-7.

## Revendications

1. Protéine qui, en tant que protéine mature active/peptide mature actif, présente la séquence suivante :
a) SEQ ID n° 1 (SAP-2)
ou
b) qui, en tant que protéine mature active, comporte des modifications alléliques de la séquence d'aminoacides mentionnée précédemment en a), jusqu'à 30 aminoacides de la séquence d'aminoacides étant remplacés, supprimés ou insérés,
l'activité antimicrobienne et/ou antibiotique de la protéine mature active définie en a) étant conservée.

2. Protéine qui comprend une séquence signal et une protéine mature selon la revendication 1,
la protéine présentant la séquence suivante :
a) SEQ ID n° 2 (PreSAP-2)
ou
b) la protéine comportant des modifications alléliques de la séquence d'aminoacides mentionnée précédemment en a), jusqu'à 35 aminoacides de la séquence d'aminoacides étant remplacés, supprimés ou insérés,
l'activité antimicrobienne et/ou antibiotique de la protéine mature active définie en a) étant conservée.

3. Protéine selon l'une quelconque des revendications précédentes, dans laquelle des groupes protecteurs sont placés à l'extrémité N-terminale et/ou à l'extrémité C-terminale.

4. Protéine selon l'une quelconque des revendications précédentes, qui est une protéine recombinante.

5. ADNc ou ADN,
l'ADNc ou l'ADN codant la séquence d'aminoacides suivante :
aa) SEQ ID n° 1 (SAP-2) ou
SEQ ID n° 2 (PreSAP-2) ;
ou
bb) l'ADNC ou l'ADN codant des modifications alléliques d'une des séquences d'aminoacides en aa), dans laquelle jusqu'à 30 aminoacides (SAP-2) ou 35 aminoacides (PreSAP-2) de la séquence d'aminoacides sont remplacés, supprimés ou insérés, l'activité antimicrobienne et/ou antibiotique de la protéine mature active définie en a) étant conservée.

6. ADNc ou ADN selon la revendication 5, l'ADNc ou l'ADN codant pour une protéine mature.

7. ADNc selon la revendication 5 ou 6,
constitué d'une des séquences nucléotidiques suivantes :
aa) SEQ ID n° 3 (ADNc-SAP-2) ou
SEQ ID n° 4 (ADNc-PreSAP-2) ;
ou
bb) l'ADNC ou l'ADN codant des modifications alléliques d'une des séquences nucléotidiques en aa), dans laquelle jusqu'à 90 nucléotides sont remplacés, supprimés ou insérés, l'activité antimicrobienne et/ou antibiotique de la protéine qui est codée par la modification allélique d'une des séquences nucléotidiques en aa) étant conservée.

8. Vecteur qui contient un ADNc ou un ADN selon l'une quelconque des revendications 5 à 7, ainsi qu'un promoteur et éventuellement un enhancer.

9. Cellule hôte eucaryote ou procaryote, qui est transformée par le vecteur selon la revendication 8.

10. Protéine selon l'une quelconque des revendications 1 à 4, pour utilisation en tant que substance active pharmaceutique.

11. Composition pharmaceutique qui contient une des protéine selon l'une quelconque des revendications 1 à 4 ou un mélange de celles-ci, en présence de composés et véhicules pharmaceutiquement acceptables et compatibles.

12. Utilisation d'une des protéines selon l'une quelconque des revendications 1 à 4, ou d'un mélange de celles-ci, pour la fabrication d'un médicament destiné au traitement d'infections qui ont été provoquées par des micro-organismes ou à la prévention de telles infections.

13. Procédé pour la synthèse d'une des protéines selon l'une quelconque des revendications précédentes 1 à 4, dans lequel les protéines sont synthétisées selon la méthode en phase solide ou selon la méthode en phase liquide.

14. Procédé pour la production d'anticorps monoclonaux
ou de fragments d'anticorps monoclonaux, avec utilisation d'au moins une protéine selon l'une quelconque des revendications 1 à 14.

15. Anticorps ou fragments des anticorps, qui reconnaissent spécifiquement des domaines sur la protéine mature selon l'une quelconque des revendications précédentes 1 à 4.

16. Anticorps ou fragments des anticorps, qui reconnaissent spécifiquement des domaines sur la protéine mature selon l'une quelconque des revendications 1 à 4, pour utilisation en tant qu'agent de diagnostic.

17. Pansement
comportant au moins une protéine selon l'une quelconque des revendications précédentes 1 à 4
ou
cellules humaines syngènes ou allogènes, qui sont transfectées avec de l'ADNc selon l'une quelconque des revendications 5-7.
